# EUROPEAN PATENT SPECIFICATION

(11) **EP 4 331 660 B1**
(45) Date of publication and mention of the grant of the patent: **15.04.2026**
(21) Application number: 23192064.6
(22) Date of filing: 18.08.2023
(51) Int. Cl.: A61N 1/36, A61N 1/378, H04R 25/00

(54) **HEARING IMPLANT AND HEARING SYSTEM**
HÖRIMPLANTAT UND HÖRSYSTEM
IMPLANT AUDITIF ET SYSTÈME AUDITIF

(30) Priority: 29.08.2022 DK PA202200796
(43) Date of publication of application: 06.03.2024
(73) Proprietor: GN Hearing A/S, 2750 Ballerup (DK)
(72) Inventor: LØNGAA, Michael, 2750 Ballerup (DK); PEDERSEN, Brian Dam, 2750 Ballerup (DK)
(74) Representative: GN Store Nord A/S

(56) References cited:
- WO-A1-2022/074479
- WO-A1-2022/162582
- WO-A1-2023/021487
- WO-A1-2023/105357

## Description

### FIELD OF THE INVENTION

The present invention relates to a hearing implant, an external hearing device and a hearing system wherein the hearing implant is implantable in a user. The hearing implant comprises an electrode array for stimulating a cochlea nerve of the user and a rechargeable battery assembly comprising a rechargeable battery. The rechargeable battery is chargeable by power or energy supplied by an external charging device via a wireless magnetic induction link.

### BACKGROUND OF THE INVENTION

Current hearing implants for stimulating the cochlea of a user are driven by a magnetic induction (MI) coil implanted under the skin on the skull of the user. During use, an external speech processing unit arranged behind the ear of the user and wired to an external MI coil arranged on the head of the user opposite the MI coil of the implant will wirelessly transfer both coded signals representative of stimulation signals and electrical power via near-field magnetic coupling between the external MI coil to the MI coil of the implant. An electrode array in the implant is then supplied with the coded signals and processes the coded signals into stimulating output signals, stimulating the cochlea nerve of the user accordingly. The magnetic coupling between the external MI coil and internal MI coil is required during normal operation of the hearing system. The presence of the external MI coil attached or fixed to the user's skin proximately to the hearing implant makes the external MI coil visible for others. Furthermore, the external MI coil is typically held in place and aligned with the MI coil of the implant by means of cooperating magnets on each side of the user's skin. Consequently, if the magnets are too strong that may cause damage, infection and/or irritation of the user's skin between the two coils and worst-case exposing the implant. On the other hand, if the cooperating magnets are too weak the external MI coil may fall off and often disconnecting the MI coil of the implant from the external MI coil. Thereby disconnecting the electrode array from the microphones on the speech processing unit such as an external hearing device.

It common practice after fitting the hearing implant and hearing system the user starts wearing the external MI coil for only a few hours per day. Wearing times can should then be gradually increased to allow the skin to adapt to the pressure from the magnet(s). If any irritation is noted the external MI coil should be removed in order to let the skin rest.

Hence, an improved hearing implant and a corresponding hearing system would be advantageous. In particular, an external hearing device, and corresponding hearing system, where the above-mentioned skin-attached or fixed external MI coil is eliminated. The elimination of the skin-attached or fixed external MI coil leads to numerous user benefits by removing at least some of the disadvantages outlined above. WO 2023/105357 A1 and WO 2022/162582 A1 provide a general architecture of an implantable hearing system using inductive coupling for both data and power transfer.

### OBJECT OF THE INVENTION

It is an objective of the present innovation to overcome the above-presented limitations in the prior art. In particular, it is an objective to provide a hearing implant and a hearing system that is less visible and avoids various inconveniences during normal use.

It is a further object of the present invention to provide a hearing implant and a hearing system with less risk of damaging the skin of the user.

It is yet a further object of the present invention to provide a more stable wireless connection between a detected microphone signal and the hearing implant.

A further object of the present invention is to provide a hearing implant and a hearing system with a faster and more convenient fitting procedure where the user does not need to wait a week or two before being able to wear it a full day.

It is a further object of the present invention to provide an alternative to the prior art.

### SUMMARY OF THE INVENTION

Thus, the above-described object and several other objects may be obtained in a first aspect of the invention by providing a hearing implant, the hearing implant being implantable in a user. The hearing implant comprises:
a first radio system which comprises a first MI coil antenna for wireless communication with an external hearing device via a NFMI data communication link,
a rechargeable battery assembly comprising a rechargeable battery and an receiver (Rx) charging coil configured for supplying charging power to the rechargeable battery. The Rx charging coil is connectable to a transmitter (Tx) charging coil of an external charging device via a wireless magnetic induction link for receipt of the charging power from the external charging device.

The hearing implant further comprises:
an electrode array for stimulating a cochlea nerve of the user in response to received electric stimulation signals,
a first control unit configured for receiving a first digital signal via the first radio system, and for processing the first digital signal into the electric stimulation signals where the first digital signal is representative of an acoustic signal, such as an audio signal like speech, detected and/or received by the external hearing device.

The NFMI data communication link preferably comprises physical layer that communicates by wirelessly coupling a non-propagating magnetic field between a transmitter coil antenna, such as the first MI coil antenna, of a first device, such as the hearing implant, and a receiver coil antenna of a second device such as the external hearing device.

The external charging device may generate a square wave or other switching waveform for transmission through the wireless magnetic induction link to the hearing implant. The frequency of the square wave or other switching waveform may be at least 10 times smaller than a carrier frequency of the NFMI data communication link. The frequency of the square wave or other switching waveform may in exemplary embodiments lie between 100 kHz and 500 kHz such as 333 kHz. The external hearing device and/or external charging device may be non-implantable and therefore external to the user's body.

The second control unit may comprise a digital signal processor (DSP) and/or a microprocessor. The second control unit may be a hybrid processor comprising a digital signal processor (DSP) and a microprocessor.

The first and/or second radio system may be configured for wireless communication/streaming, such as wireless communication/streaming between the first and second radio system.

By stimulating the cochlea nerve with a suitable stimulation output signal representative of acoustic signals such as audio signals like speech, noise etc., a hearing-impaired user may experience a sense of hearing the acoustic signal. The acoustic signal may for instance be detected and/or recorded by one or more first microphones in the external hearing device, or may be represented by a wireless signal from an external device such as a smartphone, etc. for streaming of various types of real-time or recorded audio signals.

The hearing implant is particularly non-obtrusive for the user, when compared to hearing implants known in the art. In particular, the absence of the above-discussed external and visible skin-attached magnetic inductance coil for transmitting digital signals, such as coded audio signals, and charging power to the hearing implant.

In this way, the user may achieve the convenience of a hearing implant being configured for cordless reception of the first digital signal, wherein the first digital signal is representative of an acoustic signal such as an audio signal like sound such speech and noise etc. detected and/or received by the external hearing device.

In an embodiment, the first control unit may be configured for receiving the first digital signal from the external hearing device. The first digital signal may be transmitted by the external hearing device and/or received by the hearing implant. The first digital signal may comprise data packets comprising respective segments of the first audio signal or first audio data.

The first digital signal may be detected as an acoustic signal by the one or more first microphones of the external hearing device. The one or more first microphones may convert the acoustic signal into a first electric input signal. The first digital signal may be received as a wireless signal by a second radio system in the external hearing device. The second radio system may convert the wireless signal into a second electric input signal. The second control unit may process the first and/or second electric input signal into the first digital signal and include it in the second electric output signal. The second radio system may convert the second electric output signal into a first wireless output signal. The first wireless output signal may be streamed from the second radio system to the first radio system. The first radio system may be configured to receive the first wireless output signal and convert it into the third electric input signal. The first control unit may be configured for processing the third electric input signal into the electric stimulation signal. The first control unit may be configured for processing the third electric input signal, i.e. the first digital signal into a coded signal. The coded signal may comprise a plurality of stimuli channels, such as 10 to 30 stimuli channels, such as 20 to 25 stimuli channels, such as 22 stimuli channels. The electrode array may be configured for converting the electric stimulation signal into a stimulation output signal. The electrode array may then stimulate the user's cochlea nerve with the stimulation output signal.

In this way, the first control unit may generate the electric stimulation signals from the first digital signal as received from the external hearing device. An advantage of the first digital signal being a coded signal is that the size of the audio data, such as the size of the first audio data packets, to be transmitted to and/or received by the hearing implant, is not dependent on the number of stimuli channels in the coded signal. The first digital signal may comprise compressed or uncompressed audio data.

The second control unit may provide the first compressed audio data. The second control unit may process the first and/or second electric input signal into the first compressed audio data and include it in the second electric output signal. The effect of compressing the first digital signal is that the audio data transmitted to the hearing implant is reduced in size. The advantages of transmitting compressed audio data compared to uncompressed audio data is reduced bandwidth of the first and second radio systems or more audio data can be transmitted to the hearing implant for a particular bandwidth. A further advantage is that power consumption per transmitted bit, byte of the audio data between the external hearing device and/or hearing implant may be reduced.

In an embodiment, the first control unit may be configured for receiving a first coded signal representative of the electric stimulation signals from the external hearing device. The first coded signal may be transmitted by the external hearing device and/or received by the hearing implant as a first coded stream.

The first coded signal may be detected as an acoustic signal by the one or more first microphones of the external hearing device. The one or more first microphones may convert the acoustic signal into a first electric input signal. The first coded signal may be received as a wireless signal by a second radio system in the external hearing device. The second radio system may convert the wireless signal into a second electric input signal. The second control unit may process the first and/or second electric input signal into the first coded signal and include it in the second electric output signal. The first coded signal may comprise a plurality of stimuli channels, such as 10 to 30 stimuli channels, such as 20 to 25 stimuli channels, such as 22 stimuli channels. The second radio system may convert the second electric output signal into a first wireless output signal. The first wireless output signal may be streamed from the second radio system to the first radio system. The first radio system may be configured to receive the first wireless output signal and convert it into the third electric input signal. The first control unit may be configured for processing the third electric input signal into the electric stimulation signal. The electrode array may be configured for converting the electric stimulation signal into a stimulation output signal. The electrode array may then stimulate the user's cochlea nerve with the stimulation output signal.

An advantage of transmitting a first coded signal representative of the electric stimulation signals from the external hearing device to the hearing implant, is that processing of the first digital signal by the hearing implant is less power consuming.

NFMI communication, for example using the NMFI data communication link, is particularly suitable for transmitting data between the hearing implant and the external hearing device, due to the short range of transmission requirement. NFMI communication is also attractive due to good transmission properties of NFMI communication through human tissue relative to other transmission technologies. NFMI communication is typically robust in terms of communication reliability and has low power consumption which are further advantageous properties. Finally, communication latency in NFMI communication may be reduced compared to other types of wireless communication technologies.

The NMFI data communication link may be a bidirectional wireless communication link or a unidirectional NMFI data communication link. Each of the first and second MI coil antennas may comprise a magnetic core and a coil. The coil may be coiled around the magnetic core as discussed in further detail below with reference to the appended drawings.

In an embodiment the first control unit comprises a digital signal processor (DSP) and/or a microprocessor for example software programmable processors. The first control unit may be a hybrid processor comprising a digital signal processor (DSP) and a microprocessor.

In an embodiment, the first radio system uses a carrier frequency for wireless data communication at about 5 MHz to about 30 MHz, such as about 10 MHz to about 27 MHz, or even about 20 MHz to about 25 MHz, such as 22.6 MHz.

It is an advantage to use frequencies below 30 MHz for low power short range communication as these frequencies generally are not reserved for a particular type of communication. It is an advantage to use 22.6 MHz as this frequency can be used in all countries without any requirement to apply for permission.

In an embodiment, the rechargeable battery assembly further comprises the receiver (Rx) charging coil which is configured for receiving power via the wireless magnetic induction link for charging the rechargeable battery from the external charging device. In this way, e.g. the user may conveniently charge the rechargeable battery of the hearing implant by positioning the external charging device in proximity to the Rx charging coil of the hearing implant, for example in a distinct operational mode of the hearing system. The user may for instance choose to charge the hearing implant while sleeping or relaxing as discussed in further detail below with reference to the appended drawings.

The receiver (Rx) charging coil may comprise a multi-layer air coil abutted to magnetically permeable support such as a flat circular ferrite support.

A second aspect of the invention relates to an external hearing device for mounting at, or in, a user's ear, comprising:
- a second radio system for wireless communication with the first radio system of the hearing implant via the NFMI data communication link,
wherein said second radio system comprises:
- a second MI coil antenna mounted inside a housing of the hearing device,
- a second control unit configured to generate and transmit the first digital signal to the first radio system of the hearing implant via the second MI coil antenna.

The external hearing device may comprise the one or more first microphone arranged inside the housing of the external hearing device, or e.g. connectable to the external hearing device via a dedicated wireless communication link, for pick-up of the acoustic signal from a surrounding environment of the external hearing device.

A third aspect of the invention relates to a hearing system. The hearing system at least comprises the hearing implant according to any one of the above-mentioned embodiments thereof and the external hearing device according to any of the above-mentioned embodiments of the external hearing device. The hearing system may comprise the above-mentioned external charging device The external charging device may comprise a power supply for supply of the charging power through the wireless magnetic induction link. The wireless magnetic induction link preferably comprises the Tx charging coil. In embodiments of the hearing system, the rechargeable battery assembly of the hearing implant comprises a first magnetic component and the external charging device comprises a second magnetic component. The first and second magnetic components are preferably arranged for providing magnetic attraction and alignment between the Rx charging coil and the Tx charging coil. In an embodiment, the power supply comprises a rechargeable battery. In this way providing the convenience of cordless charging of the hearing implant as the external charging device is a self-contained power source.

The external charging device may additionally comprise:
- one or more second microphone(s) for detecting an acoustic signal such as an audio signal,
- a third control unit for producing a second digital signal representative of the acoustic signal detected by the one or more second microphone(s), and
- a third radio system for communication with the first radio system of the hearing implant, the third radio system being adapted for transmitting the second digital signal to the hearing implant.

The second radio system may be configured for unidirectional or bidirectional wireless communication with the first radio system for example using the NFMI data communication link. In that system embodiment the external hearing device either detects or records an acoustic signal, such as the audio signal like sound, via the one or more first microphones, or receives a wireless signal, such as an audio signal, from an external device. The external device may for instance be a smartphone, or a tablet, or another external hearing device, etc.

If the wireless communication link between the hearing implant and external device is bidirectional, the hearing implant may transmit, e.g., control data and/or status data back to the external device via the bidirectional wireless communication link. Such return signal may also be used for troubleshooting, calibration, setup, etc. As an example, the return signal may be a mirror signal of the stimulation output signal to allow hearing care professionals to listen in on the stimulation output signal received by the user.

In an embodiment, the rechargeable battery assembly and/or the external charging device further comprises a first and/or second magnetic component arranged for providing magnetic attraction and alignment between the Rx charging coil and the Tx charging coil.

In an embodiment, the external charging device further comprises: one or more second microphone(s) for detecting an acoustic signal, a third control unit for producing a second digital signal representative of the acoustic signal detected by the one or more second microphone(s), and a third radio system for communication with the first radio system of the hearing implant, the third radio system being adapted for transmitting the second digital signal to the hearing implant.

The second digital signal may be transmitted instead of the first digital signal for example while charging the hearing implant and/or the user does not wear the external hearing device. The third radio system may comprise a third near-field magnetic inductance (MI) coil. The third control unit may comprise a digital signal processor (DSP) and/or a microprocessor. The third control unit may be a hybrid processor comprising a digital signal processor (DSP) and a microprocessor.

The first and/or third radio system may be configured for wireless communication for example streaming of audio data such as wireless communication between the first and third radio system. The third radio system may be configured for unidirectional or bidirectional wireless communication with the first radio system.

The second digital signal may comprise a fourth electric output signal provided by the third control unit comprised in the external charging device and/or a second wireless output signal provided by the third radio system comprised in the external charging device and/or the third electric input signal provided by the first radio system comprised in the hearing implant.

In this way, the user may receive acoustic signal such as sound from the external charging device. For instance, it may be convenient to remove the external hearing device to exclusively use the external charging device for providing sound input in certain situations. One example could be when the user is sleeping, and would like to have a break from wearing the external hearing device, but still be able to hear sounds from the surroundings for safety or other reasons.

In an embodiment, the second digital signal may comprise second audio data representative of an acoustic signal recorded/detected by the one or more second microphones of the external charging device. The second audio data signal may comprise audio signals and/or data. The second audio data may be transmitted by the external charging device and/or received by the hearing implant as a second audio/data stream. The second audio data may be transmitted as second audio/data packets.

The second audio data may be recorded/detected as an acoustic signal by the one or more second microphones of the external charging device. The one or more second microphones may convert the acoustic signal into the fourth electric input signal. The third control unit may process the fourth electric input signal into the second audio data and include it in the fourth electric output signal. The third radio system may convert the fourth electric output signal into the second wireless output signal. The second wireless output signal may be streamed from the third radio system to the first radio system. The first radio system may be configured to receive the second wireless output signal and convert it into the third electric input signal. The first control unit may be configured for processing the third electric input signal into the electric stimulation signal. The first control unit may be configured for processing the third electric input signal, i.e. the second audio data, into a coded signal. The coded signal may comprise a plurality of stimuli channels, such as 10 to 30 stimuli channels, such as 20 to 25 stimuli channels, such as 22 stimuli channels. The electrode array may be configured for converting the electric stimulation signal into a stimulation output signal. The electrode array may then stimulate the user's cochlea nerve with the stimulation output signal.

In this way, the first control unit may generate the electric stimulation signals from the second audio data as received from the external charging device.

An advantage of the second digital signal being second audio data is that the size of the audio data, such as the size of the second audio data packets, to be transmitted to and/or received by the hearing implant is not dependent on the number of stimuli channels in the coded signal.

In an embodiment, the second audio data is a second compressed audio data. The second audio data may be compressed or uncompressed. The third control unit may provide a second compressed audio data. The third control unit may process the fourth electric input signal into the second compressed audio data and include it in the fourth electric output signal. The effect of compressing the second audio data is that the audio/data to be transmitted to the hearing implant is reduced in size. The advantages of transmitting a second compressed audio data compared to a second uncompressed audio data is that lower capacity/bandwidth first and third radio systems may be used or that more audio/data may be transmitted to the hearing implant. A further advantage is that power consumption in the external charging device and/or hearing implant may be reduced per transmitted audio/data.

In an embodiment, the second digital signal may comprise a second coded signal representative of an electric stimulation signal. The second coded signal may be transmitted by the external charging device and/or received by the hearing implant as a second coded stream.

The second coded signal may be detected as an acoustic signal by the one or more second microphones of the external charging device. The one or more second microphones may convert the acoustic signal into the fourth electric input signal. The third control unit may process the fourth electric input signal into the second coded signal and include it in the fourth electric output signal. The second coded signal may comprise a plurality of stimuli channels, such as 10 to 30 stimuli channels, such as 20 to 25 stimuli channels, such as 22 stimuli channels. The third radio system may convert the fourth electric output signal into the second wireless output signal. The second wireless output signal may be streamed from the third radio system to the first radio system. The first radio system may be configured to receive the second wireless output signal and convert it into the third electric input signal. The first control unit may be configured for processing the third electric input signal into the electric stimulation signal. The electrode array may be configured for converting the electric stimulation signal into a stimulation output signal. The electrode array may then stimulate the user's cochlea nerve with the stimulation output signal.

An advantage of transmitting a second coded signal representative of the electric stimulation signals from the external charging device to the hearing implant, is that processing of the second digital signal by the hearing implant is less power consuming.

The user may wear two external hearing devices, one hearing device at each ear. The two hearing devices may be connected, such as wirelessly connected and/or connected by wires, such as a binaural hearing aid system. The hearing device, such as the external hearing device, may be a hearable such as an earbud, hearing aid, a personal sound amplification product (PSAP), an over-the-counter (OTC) hearing device, a one-size-fits-all hearing device, a custom hearing device or another head-wearable hearing device. Hearing devices can include both prescription devices and non-prescription devices.

The external hearing device may comprise a housing supporting, enclosing and protecting various types of electronic component and input and output transducers like one or more of the second radio system, the second control unit, the second MI coil antenna etc. The external hearing device may be embodied in various housing styles or form factors. Some of these form factors are Behind-the-Ear (BTE) hearing device or Receiver-in-Canal (RIC) hearing device also known as Receiver-in-Ear (RIE) hearing device or Microphone-and-Receiver-in-Ear (MaRIE) hearing device. These devices may comprise a behind the ear (BTE) unit configured to be worn behind the ear of the user and an in the ear (ITE) unit configured to be inserted partly or fully into the user's ear canal. Generally, the BTE unit may comprise at least one input transducer, such as the one or more first microphones, a power source and a processing unit, such as the second control unit. The term BTE hearing device refers to a hearing device where the receiver, i.e. the output transducer, is comprised in the BTE unit and sound is guided to the ITE unit via a sound tube connecting the BTE and ITE units, whereas the terms RIE, RIC and MaRIE hearing devices refer to hearing devices where the output transducer may be comprise in the ITE unit, which is coupled to the BTE unit via a connector cable or electrical wire/tube configured for transferring electric signals between the BTE and ITE units. The output transducer may be omitted or disabled.

Some of these form factors are In-the-Ear (ITE) hearing device, Completely-in-Canal (CIC) hearing device or Invisible-in-Canal (IIC) hearing device. These hearing devices may comprise an ITE unit, wherein the ITE unit may comprise at least one input transducer, such as the one or more first microphones, a power source, a processing unit, such as the second control unit, and an output transducer. The output transducer may be omitted or disabled. These form factors may be custom devices, meaning that the ITE unit may comprise a housing having a shell made from a hard material, such as a hard polymer or metal, or a soft material such as a rubber-like polymer, molded to have an outer shape conforming to the shape of the specific user's ear canal.

The person skilled in the art is well aware of different kinds of hearing devices and of different options for arranging the hearing device in, on, over and/or at the ear of the hearing device wearer. The hearing device, such as the external hearing device, (or pair of hearing devices) may be custom fitted, standard fitted, open fitted and/or occlusive fitted.

In an embodiment, the hearing device, such as the external hearing device, may comprise the one or more input transducers such as one or more microphones, such as the one or more first microphone(s). The one or more input transducers may comprise one or more vibration sensors configured for detecting bone vibration. The one or more input transducer(s) may be configured for converting an acoustic signal into a first electric input signal. The first electric input signal may be an analogue signal. The first electric input signal may be a digital signal. The one or more input transducer(s) may be coupled to one or more analogue-to-digital converter(s) configured for converting the analogue first input signal into a digital first input signal.

In an embodiment, the hearing device, such as the external hearing device, may comprise one or more antenna(s) configured for wireless communication. One of the one or more antenna(s) may be part of the second radio system. The one or more antenna(s) may comprise an electric antenna. The electric antenna may be configured for wireless communication at a first frequency. The first frequency may be above 800 MHz, preferably a wavelength between 900 MHz and 6 GHz. The first frequency may be 902 MHz to 928 MHz. The first frequency may be 2.4 to 2.5 GHz. The first frequency may be 5.725 GHz to 5.875 GHz. The one or more antenna(s) may comprise a magnetic antenna, i.e. MI coil antenna as discussed above.

In an embodiment, the hearing device, such as the external hearing device, may comprise one or more wireless communication unit(s). One of the one or more wireless communication unit(s) may be part of the second radio system. The one or more wireless communication unit(s) may comprise one or more wireless receiver(s), one or more wireless transmitter(s), one or more transmitter-receiver pair(s) and/or one or more transceiver(s). At least one of the one or more wireless communication unit(s) may be coupled to the one or more antenna(s). The wireless communication unit may be configured for converting a wireless signal received by at least one of the one or more antenna(s) into a second electric input signal. The hearing device may be configured for wired/wireless audio communication, e.g. enabling the user to listen to media, such as music or radio and/or enabling the user to perform phone calls.

In an embodiment, the wireless signal may originate from one or more external source(s) and/or external devices, such as spouse microphone device(s), wireless audio transmitter(s), smart computer(s) and/or distributed microphone array(s) associated with a wireless transmitter. The wireless input signal(s) may origin from another hearing device, such as another or second external hearing device, e.g., as part of a binaural hearing system and/or from one or more accessory device(s), such as a smartphone and/or a smart watch.

In an embodiment, the hearing device, such as the external hearing device, may include a processing unit, such as the second control unit. The processing unit may be configured for processing the first and/or second electric input signal(s). The processing may comprise compensating for a hearing loss of the user, i.e., apply frequency dependent gain to input signals in accordance with the user's frequency dependent hearing impairment. The processing may comprise performing feedback cancelation, beamforming, tinnitus reduction/masking, noise reduction, noise cancellation, speech recognition, bass adjustment, treble adjustment and/or processing of user input. The processing unit may be a processor, an integrated circuit, an application, functional module, etc. The processing unit may be implemented in a signal-processing chip or a printed circuit board (PCB). The processing unit may be configured to provide a first electric output signal based on the processing of the first and/or second electric input signal(s). The processing unit may be configured to provide a second electric output signal. The second electric output signal may be based on the processing of the first and/or second electric input signal(s).

In an embodiment, the hearing device, such as the external hearing device, may comprise an output transducer. The output transducer may be coupled to the processing unit. The output transducer may be a receiver. It is noted that in this context, a receiver may be a loudspeaker, whereas a wireless receiver may be a device configured for processing a wireless signal. The receiver may be configured for converting the first electric output signal into an acoustic output signal. The output transducer may be coupled to the processing unit via the magnetic antenna. The output transducer may be comprised in an ITE unit or in an earpiece, e.g. Receiver-in-Ear (RIE) unit or Microphone-and-Receiver-in-Ear (MaRIE) unit, of the hearing device. One or more of the input transducer(s) may be comprised in an ITE unit or in an earpiece. The output transducer may be omitted or disabled.

In an embodiment, the wireless communication unit, such as the second radio system, may be configured for converting the second electric output signal into a wireless output signal, such as the first wireless output signal. The wireless output signal may comprise synchronization data. The wireless communication unit may be configured for transmitting the wireless output signal via at least one of the one or more antennas.

In an embodiment, the hearing device, such as the external hearing device, may comprise a digital-to-analogue converter configured to convert the first electric output signal, the second electric output signal and/or the wireless output signal, such as the first wireless output signal, into an analogue signal.

In an embodiment, the hearing device, such as the external hearing device, may comprise a vent. A vent is a physical passageway such as a canal or tube primarily placed to offer pressure equalization across a housing placed in the ear such as an ITE hearing device, an ITE unit of a BTE hearing device, a CIC hearing device, a RIE hearing device, a RIC hearing device, a MaRIE hearing device or a dome tip/earmold. The vent may be a pressure vent with a small cross section area, which is preferably acoustically sealed. The vent may be an acoustic vent configured for occlusion cancellation. The vent may be an active vent enabling opening or closing of the vent during use of the hearing device. The active vent may comprise a valve.

In an embodiment, the hearing device, such as the external hearing device, may comprise a power source. The power source may comprise a battery providing a first voltage. The battery may be a rechargeable battery. The battery may be a replaceable battery. The power source may comprise a power management unit. The power management unit may be configured to convert the first voltage into a second voltage. The power source may comprise a charging coil. The charging coil may be provided by the magnetic antenna.

In an embodiment, the hearing device, such as the external hearing device, may comprise a memory, including volatile and non-volatile forms of memory.

A fourth aspect of the invention relates to a method of operating a hearing system comprising a hearing implant, an external hearing device and an external charging device for example according to the respective embodiments described above. Said method comprising a first operational mode comprising:
- detect an acoustic signal, such as an audio signal, at the external hearing device,
- convert the acoustic signal into a corresponding first digital signal by a control unit of the external hearing device,
- transmit the first digital signal by a first MI coil antenna of the external hearing device to a second MI coil antenna of the hearing implant,
- processing the first digital signal into electric stimulation signals by a first control unit of the hearing implant,
- apply the electric stimulation signals to an electrode array of the hearing implant; and
a second operational mode comprising:
- transmit power from the external charging device to a Rx charging coil of the hearing implant via a wireless magnetic induction link,
- transfer power or energy from the Rx charging coil to a rechargeable battery of the hearing implant to charge the rechargeable battery.

The method of operating the hearing system may further comprise:
- disable the wireless magnetic induction link of the external charging device in the first operational mode; and/or
- detach the external hearing device from the user' ear, and optionally, disable the NFMI data communication link, in the second operational mode. The user may for example place the external hearing device in a battery charger or another suitable place.

The method of operating the hearing system may further comprise:
- in the second operational mode, position the Tx charging coil of the external charging device in proximity to the Rx charging coil of the external hearing device, for example with a distance of less than 5 cm, for example by use of a pair of cooperating magnets of the devices which to provide fixation and alignment between the Tx charging coil and the Rx charging coil.

The first and second aspects of the present invention may be combined. These and other aspects of the invention will be apparent from and elucidated with reference to the embodiments described hereinafter.

### BRIEF DESCRIPTION OF THE FIGURES

The hearing implant and hearing system according to the invention will now be described in more detail with regard to the accompanying figures. The figures show one way of implementing the present invention and is not to be construed as being limiting to other possible embodiments falling within the scope of the attached claim set.
Figure 1 schematically illustrates an embodiment of the hearing implant of an exemplary hearing system according to the invention.
Figure 2 schematically illustrates the hearing system in a first operational mode.
Figure 2A schematically illustrates the hearing system in a second operational mode.
Figure 3a and 3b illustrate two embodiments of external charging devices according to exemplary embodiments of the invention.
Figure 4 illustrates an embodiment of a communication flow according to exemplary embodiments of the invention.
Figure 5 shows an exemplary MI coil antenna for use in an exemplary hearing implant of the exemplary hearing system.
Figure 6 shows an exemplary receiver (Rx) charging coil for use in an exemplary hearing implant of the exemplary hearing system.
Figure 7 shows an exemplary transmitter (Tx) charging coil for use in an exemplary external charging device of the exemplary hearing system.

### DETAILED DESCRIPTION OF EMBODIMENTS

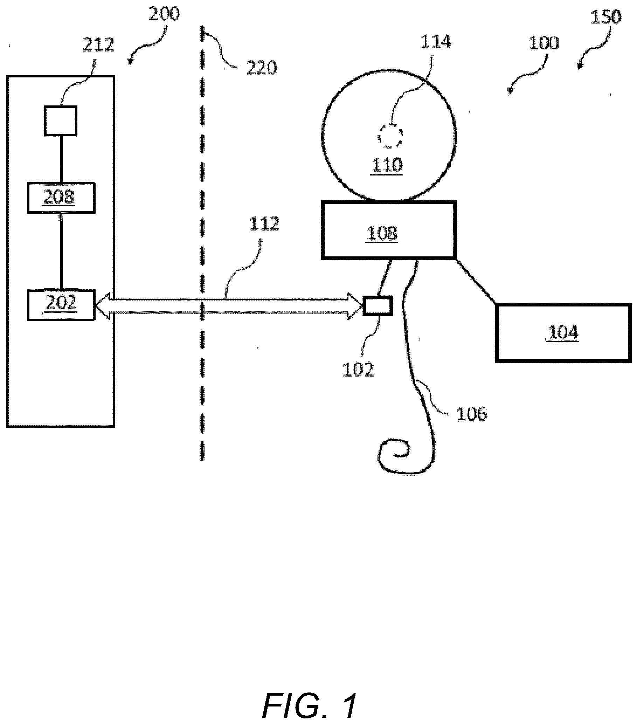

Figure 1 illustrates a hearing implant 100 according to an embodiment of the invention. Figure 1 further illustrates an external hearing device 200. The hearing implant 100 and the external hearing device 200 together form an embodiment of a hearing system 150 according to an exemplary embodiment of the invention. Dashed line 220 illustrates the skin barrier of the user, meaning that the hearing implant 100 is arranged subcutaneously in the user's head. The hearing implant 100 may be surgically implanted in the user's skull while the external hearing device 200 is external to the user, i.e. not implanted. The external hearing device 200 is typically shaped and sized for mounting at the user's ear for example arranged behind the user's ear and/or at least partly arranged in the user's ear canal. The hearing implant 100 comprises a first radio system 102 for wireless communication with a second radio system 202 of the external hearing device 200 via a wireless communication link 112 such as a near-field magnetic induction (NFMI) based data communication link. The wireless communication link 112 may be a bidirectional wireless communication link. The first radio system 102 is connected to a first control unit 108. In some embodiments, the first control unit 108 may be a processing unit such as a digital signal processor (DSP) and/or a microprocessor.

The hearing implant 100 further comprises a rechargeable battery assembly 104 that inter alia comprises a rechargeable battery. The rechargeable battery assembly 104 is configured for receiving electrical power from a receiver (Rx) charging coil 110 during a second operational mode, i.e. a charging mode, of the hearing system 50 as discussed in further detail below. During a first operational mode of the hearing system the wireless communication link 112 is active for receipt of a first digital signal. The first digital signal may comprise audio data such as encoded acoustic signals received by the external hearing device 200. The first digital signal may comprise various type of control data and error-check and/or error-correction data in addition to the encoded acoustic signals. Only the external hearing device 200 may be visible and as such similar to the visibility of a regular hearing device/hearing aid.

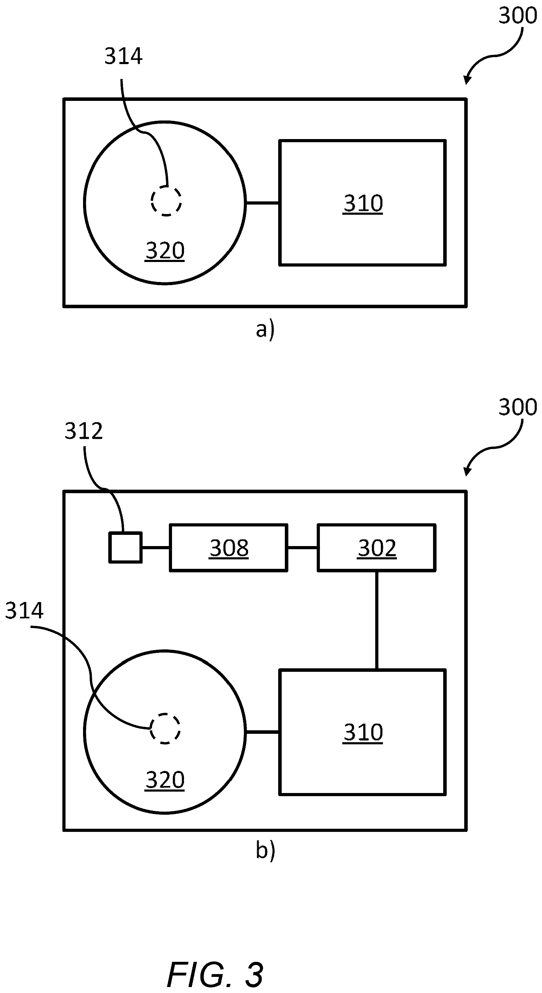

The hearing implant 150 further comprises an electrode array 106 for insertion into the cochlea of the user by implantation. The hearing implant 100 may further comprise a first magnetic component 114 like a permanent magnet configured to magnetically engage with a second magnetic component like a permanent magnet of an external charging device. A second magnetic component 314 of the external charging device 300 is schematically illustrated in figure 3a and 3b. The first magnetic component 114 may further be configured for providing magnetic fixation and alignment between the receiver (Rx) charging coil 110 and a transmitter (Tx) charging coil of an external charging device, such as the transmitter (Tx) charging coil 320 of the external charging devices 300 of figure 3a and 3b. In some embodiments, the first magnetic component 114 comprises a magnetic material, or a magnet. The magnetic material may be a metal, such as stainless steel. The magnet may be a permanent magnet, such as a permanent magnet that is ferromagnetic or ferromagnetic, or such as a material having a weaker type of magnetism, or a temporary magnet, such as an electromagnet.

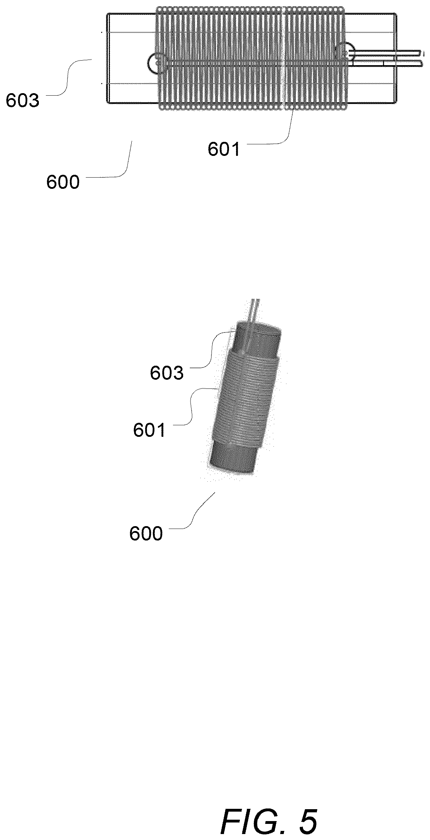

The external hearing device 200 comprises the second radio system 202 for communicating with the first radio system 102 of the hearing implant 100. The second radio system preferably comprises a second MI coil antenna (not shown on figure 1 but illustrated on Fig. 5) which is a part of the previously discussed NFMI based data communication link. The second MI coil antenna is preferably mounted inside a housing of the external hearing device 200 to render the second MI coil antenna invisible during use of the hearing system 150. The arrangement of the second MI coil antenna inside the housing of the external hearing device 200 eliminates the traditional above-discussed visible and skin-attached external MI coil of prior art cochlear implant hearing systems.

The second radio system 202 is connected to a second control unit 208 for communication of the first digital signal. The second control unit 208 may be configured to generate and transmit the first digital signal to the first radio system 102 of the hearing implant 100 via the wireless communication link 112. In some embodiments, the second control unit 208 may comprise processing unit, such as a digital signal processor (DSP) and/or a microprocessor. The second control unit 208 may comprise a transmit circuit (Tx) and optionally a receipt circuit (Rx) connected to the second MI coil antenna. The Tx circuit may be configured to modulate the first digital signal by a predetermined carrier frequency before transmission through the wireless communication link 112. In the embodiment shown here, the external hearing device further comprises one or more first microphones 212 for receipt of acoustic signals, such as audio signals like speech, noise etc., from the surrounding environment of the user. The one or more first microphones 212 is connected to the second control unit 208. The second control unit 208 may be configured to encode and add the acoustic signals to the first digital signal before transmission to the hearing implant 150 via the wireless communication link 112. The one or more first microphones 212 may be arranged on or in a behind the ear (BTE) unit and/or an in the ear (ITE) unit of the external hearing device 200. As an example, a BTE unit may comprise one or two first microphones of the one or more first microphones and/or an ITE unit may comprise one or two first microphones of the one or more first microphones.

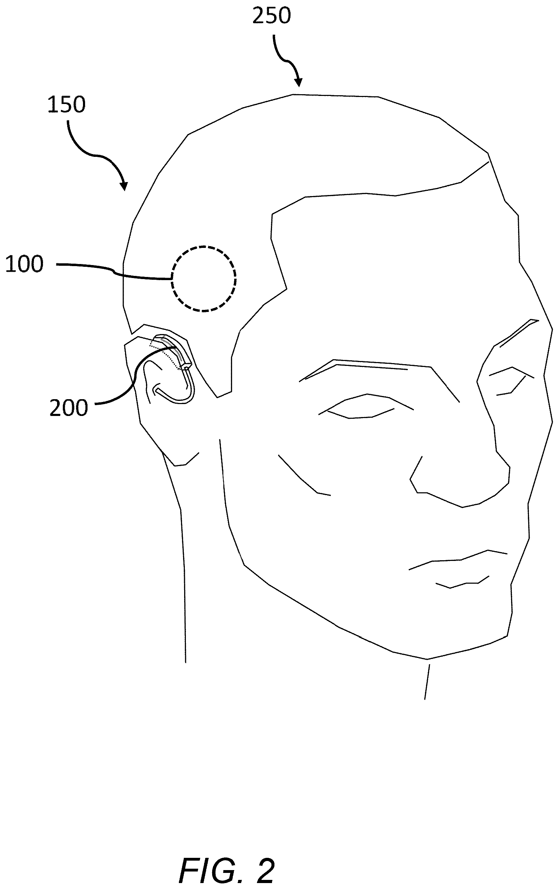

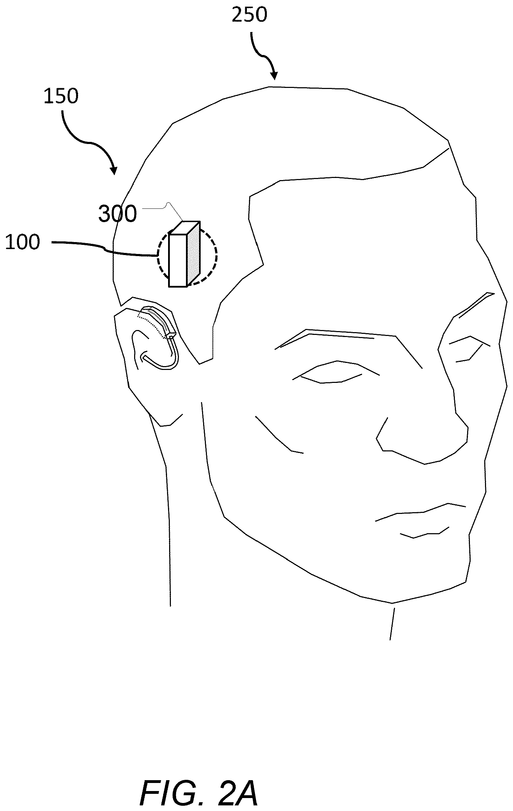

FIG. 2 illustrates a user 250 using the hearing system 150 according to an exemplary embodiment of the invention where the hearing system may operate in the first operational mode. As discussed above, in the first operational mode, the first digital signal, which typically comprises encoded audio signals as discussed above, is generated by the external hearing device 200 and transmitted to the hearing implant 100 via the NFMI based data communication link. The first control unit 108 of the hearing implant 100 is configured receive the first digital signal and process or convert the first digital signal into the electric stimulation signals for application to the cochlear nerve of the user via the electrode array. During the first operational mode, visible is only the external hearing device 200 because its MI coil antenna is arranged inside the housing of the external hearing device 200. The external hearing device 200 is illustrated as a Behind-the-Ear (BTE) type hearing device, such as Behind-the-Ear (BTE) hearing device/aid, Receiver-in-Ear (RIE) hearing device/aid or Microphone-and-Receiver-in-Ear (MaRIE) hearing device/aid. Alternatively, the external hearing device 200 may be an In-the-Ear (ITE) type hearing device/aid, wherein a hearing system comprising an In-the-Ear (ITE) type hearing device/aid may be even less visible. Also indicated in the figure by the dashed circle the hearing implant 100 is located subcutaneously under the user's skin.

If the hearing implant according to embodiments of the invention is used together with a BTE type hearing device, the hook, if it is a Behind-the-Ear (BTE) hearing device/aid, or the in the ear (ITE) unit, if being a Receiver-in-Ear (RIE) hearing device/aid or Microphone-and-Receiver-in-Ear (MaRIE) hearing device/aid, may function as retention elements in order to retain a behind the ear (BTE) unit of the BTE type hearing device in place at/on/behind the user's ear. The BTE or ITE unit may or may not comprise a receiver such as a miniature loudspeaker. In case the BTE or ITE unit comprise a receiver, the receiver may be disabled, i.e. not provide any acoustic output signal to the ear it is worn by the user.

FIG. 2A illustrates a user 250 using the hearing system 150 according to the exemplary embodiment of the invention as discussed above, but where the hearing system 150 operates in the second operational mode. In the second operational mode, the user positions the external charging device 300 such that the Tx charging coil 310 of the external charging device 300 is aligned with the Rx charging coil of the external hearing device 100, for example using the previously discussed pair of cooperating magnetic component 314, 114 of the devices. The external hearing device 200 may be detached from the user's ear during the second operational mode and/or possibly be deactivated e.g. in a powered-off state. The external charging device 300 transmits power to the Rx charging coil of the hearing implant 100 via the wireless magnetic induction link that comprises the magnetically coupled Tx charging coil 310 and Rx charging coil 110. The power or energy received by the Rx charging coil 110 is transferred to the rechargeable battery of the hearing implant 150 for example through an electrical wire or trace so as to charge the rechargeable battery during the second operational mode.

In this illustrative embodiment, the hearing implant 100 is temporarily or permanently implanted in/at one ear of the user and the external hearing device 200 is configured to be worn at/on/in the same ear of the user as the hearing implant. The external hearing device 200 may be a first external hearing device worn and/or the hearing implant may be a first hearing implant implanted at/on/in one of the user's ears, wherein the hearing system may further comprise another/a second external hearing device worn and/or another/a second hearing implant, not shown, implanted at/on/in the user's other ear.

The user may wear the second external hearing device at/on/in the other ear if the other ear is hearing impaired. The user may wear the second hearing implant and the second external hearing device at/on/in the other ear if the other ear is deaf or extremely hearing impaired. The second hearing implant may have a radio system similar to, and preferably compatible with, the first radio system. The second external hearing device may have a radio system similar to, and preferably compatible with, the second radio system, wherein the second hearing implant may be configured for wireless communication with the second external hearing device. The second hearing device may further be configured for wireless communication with the first hearing device for example as part of binaural hearing system.

The second hearing implant may be similar to the first hearing implant, i.e. have the same features and advantages. The second external hearing device may be similar to the first external hearing device, i.e. have the same features and advantages. The form factor of the first and second external hearing devices may though not necessarily be the same.

FIG. 3a and 3b illustrate two different embodiments of the external charging device 300 which may form part of the previously discussed exemplary hearing systems.

The two embodiments of the external charging device 300 are related in that identical reference numerals refer to similar or identical parts. FIG. 3a shows a simple external charging device 300 for charging or recharging the hearing implant 100 for example during a dedicated charging mode of the hearing system. The external charging device 300 may comprise a power supply 310 in addition to the Tx charging coil 320. The Tx charging coil 320 may be coupled to the Rx charging coil 110 when the Rx charging coil 320 the Rx charging coil 110 are brought in proximity to each other for example fixed in predetermined position and alignment or orientation. When the wireless magnetic induction link is established the two charging coils 110, 310 obtains inductively coupling and electrical power from the power supply 310 of the external charging device 300 is transferred to the hearing implant 100 as discussed above.

To aid the user 250 in aligning the charging coils 110, 310, the hearing implant 100 and the external charging device 300, respectively, may each comprise one or more cooperating magnetic components. The one or more cooperating magnetic components may magnetically engage when the two charging coils 110, 310 align, such as the first magnetic component 114 of the hearing implant 100 in FIG. 1 and the second magnetic component 314 of the external charging device 300. In some embodiments, the second magnetic component 314 is a magnetic material, or a magnet. The magnetic material may be a metal, such as stainless steel. The magnet may be a permanent magnet, such as a permanent magnet that is ferromagnetic or ferromagnetic, or such as a material having a weaker type of magnetism, or a temporary magnet, such as an electromagnet. Furthermore, the first and second magnetic components 114, 314 may be configured to keep the external charging device 300 in place on the skin of the user 250 during the charging mode of the hearing system. The power supply 310 may comprise a power converter connected to power cord connectable mains voltage e.g. a wall outlet, or may comprise a rechargeable battery to allow for cordless charging of the hearing implant 100.

The external charging device 300 shown in FIG. 3b also comprises a similar power supply 310 and similar Tx charging coil 320 described above for FIG. 3a. This embodiment further comprises one or more second microphones 312, a third control unit 308, and a third radio system 302, which are in mutual communication. In some embodiments, the third control unit 308 may be a processing unit, such as a digital signal processor (DSP) and/or a microprocessor. This allows the user to maintain a sense of hearing by connecting the third radio system 302 of the external charging device 300 to the first radio system 102 of the hearing implant 102 even if the external hearing device 200 is turned off or non-functioning. In this figure, power from the power supply 310 is shown to be coupled to the third radio system 302, which then feeds the third control unit 308 that in turn feeds the microphone 312. It is noted that each of the units 302, 308, and 312 may also be powered individually or in another sequence from the power supply 310, without deviating from the invention.

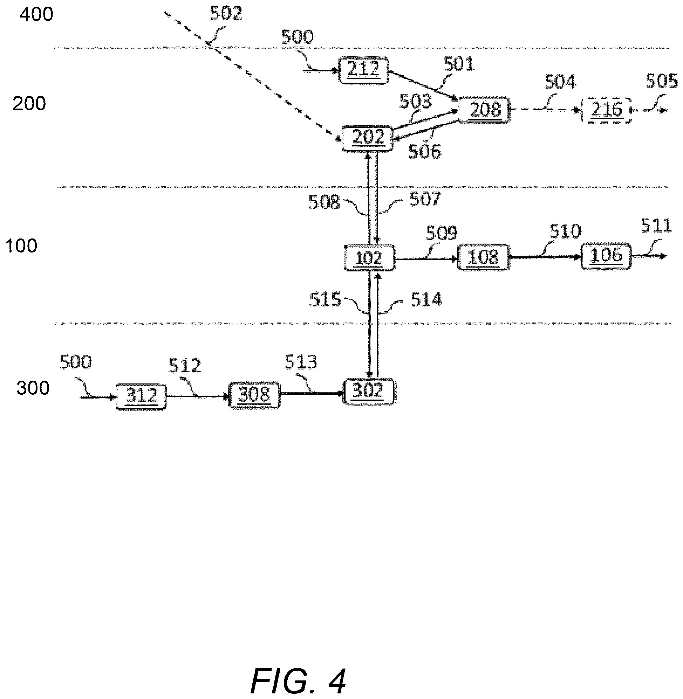

FIG. 4 illustrates an embodiment of a communication flow according to various embodiments of the exemplary hearing system . The communication flow may comprise respective wireless communication links between an external device 400 and an external hearing device 200, between the external hearing device 200 and a hearing implant 100 and between the hearing implant 100 an external charging device 300. The skilled person will understand that the exemplary hearing system may comprise a hearing implant, an external hearing device and an external charging device for example those devices discussed above while the external device 400 is optional.

One or more first microphones 212 of the external hearing device 200 may be placed and/or configured for detecting an acoustic signal 500 from the surroundings of the user and/or converting the acoustic signal 500 into a first electric input signal 501. A second radio system 202 of the external hearing device 200 may be configured for receiving a wireless signal 502 from an external device 400 and/or converting the wireless signal 502 into a second electric input signal 503. The external device 400 may for instance be a smartphone, or a tablet, or another/a second external hearing device, etc. A second control unit 208 of the external hearing device 200 may be configured for receiving the first and/or second electric input signal 501, 503 and/or processing the first and/or second electric input signal 501, 503 and/or providing a second electric output signal 506 based on the processing of the first and/or second electric input signal 501, 503. The second control unit 208 may optionally also be configured for providing a first electric output signal 504 based on the processing of the first and/or second electric input signal 501, 503. An optional receiver 216 of the external hearing device 200 may be configured for receiving the first electric output signal 504 and/or converting the first electric output signal 504 into an acoustic output signal 505 to the user and/or outputting the acoustic output signal 505. The receiver 216 may thereby provide the acoustic output signal 505 to the user. The second radio system 202 may be configured for converting the second electric output signal 506 into a first wireless output signal 507 and/or transmitting/streaming the first wireless output signal 507 via the wireless communication link 112 (shown in FIG. 1) to a first radio system 102 of the hearing implant 100. In return, the first radio system 102 may be configured to transmit/stream a first return signal 508, e.g. comprising one or more of control data, status data, calibration data, stimulation data, etc., back to the second radio system 202 via the wireless communication link 112. The first radio system 102 may be configured to receive the first wireless output signal 507 and/or convert the first wireless output signal 507 into a third electric input signal 509.

A first control unit 108 of the hearing implant 100 may be configured for receiving the third electric input signal 509 and/or processing the third electric input signal 509 into an electric stimulation signal 510. An electrode array 106 of the hearing implant 100 may be configured for receiving the electric stimulation signal 510 and/or converting the electric stimulation signal 510 into a stimulation output signal 511. The electrode array 106 may apply output the stimulation output signal 511 to the user's cochlea nerve . A first digital signal being representative of the acoustic signal 500 detected by the external hearing device 200 and/or the wireless signal 502 received by the external hearing device 200 may comprise the second electric output signal 506 provided by the second control unit 208 of the external hearing device 200 and/or the first wireless output signal 507 provided by the second radio system 208 of the external hearing device 200 and/or the third electric input signal 509 provided by the first radio system 102 of the hearing implant 100.

One or more optional second microphones 312 of the external charging device 300 may be placed and/or configured for detecting the acoustic signal 500 from the surroundings of the user and/or converting the acoustic signal 500 into a fourth electric input signal 512. A third control unit 308 of the external charging device 300 may be configured for receiving the fourth electric input signal 512 and/or processing the fourth electric input signal 512 and/or providing a fourth electric output signal 513 based on the processing of the fourth electric input signal 512. A third radio system 302 of the external charging device 300 may be configured for receiving the fourth electric output signal 513 and/or converting the fourth electric output signal 513 into a second wireless output signal 514 and/or transmitting/streaming the second wireless output signal 514 via a wireless communication link (not shown) to the first radio system 102. The wireless communication link may be a unidirectional or a bidirectional wireless communication link for example comprising a bidirectional NFMI data communication link. In return, the first radio system 102 may be configured to transmit/stream a second return signal 515, e.g., control data, status data, calibration data, stimulation data, etc., back to the third radio system 302 via the wireless communication link.

The first radio system 102 may be configured for receiving the second wireless output signal 514 and/or providing the third electric output signal 509 based on the second wireless output signal 514 to the electrode array 106. The first control unit 108 may be configured for receiving the third electric input signal 509 and/or processing the third electric input signal 509 into the electric stimulation signal 510. The electrode array 106 may be configured for receiving the electric stimulation signal 510 and/or converting the electric stimulation signal 510 into the stimulation output signal 511. The electrode array 106 may then stimulate the user's cochlea nerve with the stimulation output signal 511. A second digital signal being representative of an acoustic signal 500 detected by the external charging device 300 may comprise the fourth electric output signal 513 provided by the third control unit 308 of the external charging device 300 and/or the second wireless signal 514 outputted by the third radio system 302 of the external charging device 200 and/or the third electric input signal 509 provided by the first radio system 102 of the hearing implant 100.

FIG. 5 shows an exemplary MI coil antenna 600 for use in the exemplary hearing implant 100 of the hearing system 150. Dimensions of the first MI coil antenna 600 may be small because it is dimensioned to merely receive the first digital signal from the external hearing device 200 without receiving power for the rechargeable battery of the implant 100 from the external hearing device 200 or power from the external charging device. The first digital signal is preferably transmitted to the hearing implant 100 via the NFMI data communication link. The first MI coil antenna 600 may possess an inductance between 2 µH and 20 µH such as between 3 µH and 10 µH for example depending on the carrier frequency of the NFMI data communication link. The first MI coil antenna 600 may possess a Q value larger than 30 or 40 or 60 @10 MHz to provide large frequency selectively and good out-of-band noise rejection. As illustrated, the first MI coil antenna 600 may comprise a coil wound around a magnetically permeable core 603 such as a solenoid coil 601 wound around the magnetically permeable core 603 which may comprise a ferrite composition. The first MI coil antenna 600 may have a volume less than 20 mm³ or less than 15 mm³ irrespective of the specific shape of the first MI coil antenna 600. Certain cylindrical embodiments of the first MI coil antenna 600 has a length smaller than 6 mm and diameter smaller than 3.0 mm. The skilled person will appreciate that the dimensions and electrical characteristics of the MI coil antenna of the external hearing device may be nominally identical or similar to the MI coil antenna 600 of the exemplary hearing implant 100.

The transfer of power to the hearing implant 150 is carried out by the wireless magnetic induction link that comprises the dedicated Rx charging coil of the hearing implant 150 as well as the Tx charging coil of the external charging device 300. The wireless magnetic induction link may use a switching frequency less than the carrier frequency used by the NFMI data communication link.

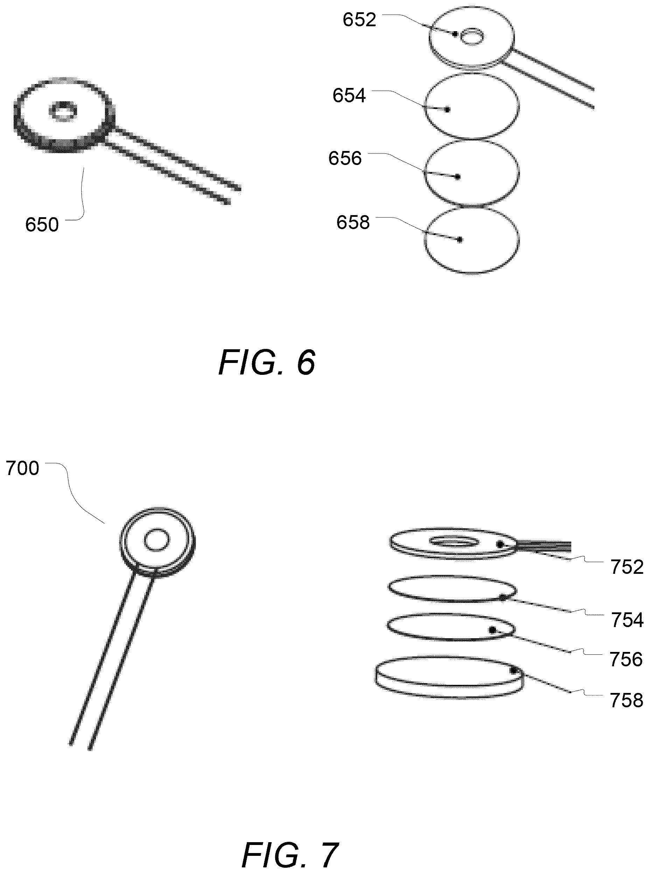

FIG. 6 shows an exemplary embodiment of the dedicated receiver (Rx) charging coil 650 suitable for use in the exemplary hearing implant 100 of the exemplary hearing system 150. An inductance of the Rx charging coil 650 may lie between 10 µH and 50 µH such as between 20 µH and 40 µH @ 100 kHz. Some embodiments of the Rx charging coil 650 comprises a multi-layer air coil 652 abutted to a magnetically permeable support 656 such as a mating flat ferrite support and fixed by an adhesive agent 654. Some embodiments of the Rx charging coil 650 may additionally comprise a second adhesive agent 658 such as an adhesive tape. The multi-layer air coil 652 may possess a circular flat shape and comprise between 20 and 100 windings for example about 60 windings. The circular flat multi-layer air coil 652 may have a diameter between 5 mm and 15 mm such as about 10 mm and a height smaller than 1 mm.

FIG. 7 shows an exemplary transmitter (Tx) charging coil 700 suitable for use in an exemplary external charging device 300 of the exemplary hearing system 150. An inductance of the Tx charging coil 750 may lie between 5 µH and 20 µH @ 100 kHz such as about 12 µH. Some embodiments of the Tx charging coil 750 comprises a multi-layer air coil 752 abutted to a magnetically permeable support 758 such as a mating flat ferrite support and fixed by an intervening adhesive agent 754 and/or a PET tape 756. The multi-layer air coil 752 may possess a circular flat shape and comprise between 20 and 50 windings for example about 30 windings. The circular flat multi-layer air coil 752 may have a diameter between 5 mm and 20 mm such as about 11 mm and a height smaller than 1 mm.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

Throughout the present disclosure the terms "first", "second", "third", "fourth", etc. are used here and elsewhere for labelling purposes only and are not intended to denote any specific spatial or temporal ordering, but are included to identify individual elements.

### REFERENCE LIST

- 100: Hearing implant
- 102: First radio system
- 104: Rechargeable battery assembly
- 106: Electrode array
- 108: First control unit
- 110: Receiver (Rx) charging coil
- 112: Wireless communication link
- 114: First magnetic component
- 150: Hearing system
- 200: External hearing device
- 202: Second radio system
- 208: Second control unit
- 212: First microphone(s)
- 216: Receiver
- 220: Skin barrier of the user
- 250: User
- 300: External charging device
- 302: Third radio system
- 308: Third control unit
- 310: Power supply
- 312: Second microphone(s)
- 314: Second magnetic component
- 320: Transmitter (Tx) charging coil
- 400: External device
- 500: Acoustic signal
- 501: First electric input signal
- 502: Wireless signal
- 503: Second electric input signal
- 504: First electric output signal
- 505: Acoustic output signal
- 506: Second electric output signal
- 507: First wireless output signal
- 508: First return signal
- 509: Third electric input signal
- 510: Electric stimulation signal
- 511: Stimulation output signal
- 512: Fourth electric input signal
- 513: Fourth electric output signal
- 514: Second wireless output signal
- 515: Second return signal
- 600: First MI coil antenna
- 650: Rx charging coil
- 700: Tx charging coil

## Claims

1. Hearing implant (100), the hearing implant (100) being implantable in a user and comprising:
- a first radio system (102) which comprises a first magnetic induction, MI coil antenna (600) for wireless communication with an external hearing device (200) via a near-field magnetic induction, NFMI data communication link (112),
- a rechargeable battery assembly (104) comprising a rechargeable battery and a receiver, Rx charging coil (650) configured for supplying charging power to the rechargeable battery, wherein the receiver, Rx charging coil (650) is connectable to a transmitter, Tx charging coil (700) of an external charging device (300) via a wireless magnetic induction link for receipt of the charging power from the external charging device (300),
- an electrode array (106) for stimulating a cochlea nerve of the user in response to received electric stimulation signals (510), and
- a first control unit (108) configured for receiving a first digital signal via the first radio system (102), and for processing the first digital signal into the electric stimulation signals (510), the first digital signal being representative of an acoustic signal, such as an audio signal like speech, detected and/or received by the external hearing device (200).

2. The hearing implant (100) according to claim 1, wherein an inductance of the first NFMI coil antenna (600) is between 2 µH and 20 µH such as between 3 µH and 10 µH.

3. The hearing implant (100) according to claim 1 or 2, wherein the first MI coil antenna (600) comprises a solenoid coil wound around a magnetically permeable core; and optionally a volume less than 20 mm³ for example a diameter smaller than 3 mm and a length smaller than 6 mm.

4. The hearing implant (100) according to claim 2 or 3, wherein an inductance of the Rx charging coil (650) is between 10 µH and 50 µH @ 100 kHz, such as between 20 µH and 40 µH.

5. The hearing implant (100) according to claim 4, wherein the receiver, Rx charging coil (650) comprises a multi-layer air coil abutted to magnetically permeable support such as a flat circular ferrite support.

6. The hearing implant (100) according to any one of the preceding claims, wherein the first control unit (108) comprises a digital signal processor, DSP and/or a microprocessor.

7. The hearing implant (100) according to any one of the preceding claims, wherein the NFMI data communication link (112) comprises a carrier frequency from about 5 MHz to about 30 MHz, such as about 10 MHz to about 27 MHz, or even about 20 MHz to about 25 MHz, such as 22.6 MHz.

8. An external hearing device (200) for mounting at, or in, a user's ear, comprising:
- a second radio system (202) for wireless communication with the first radio system (102) of the hearing implant (100) according to anyone of claims 1-7 via the NFMI data communication link (112),
wherein said second radio system (202) comprises a second MI coil antenna mounted inside a housing of the external hearing device (200),
- a second control unit (208) configured to generate and transmit the first digital signal to the first radio system (102) of the hearing implant (100) via the second MI coil antenna.

9. An external hearing device (200) according to claim 8, comprising a microphone (212) arranged inside the housing of the external hearing device (200), or connectable to the external hearing device (200), for pick-up of the acoustic signal from a surrounding environment of the external hearing device (200).

10. A hearing system (150) at least comprising:
- the hearing implant (100) according to any one of claims 1-7, and
- the external hearing device (200) according to claim 8 or 9.

11. The hearing system (150) according to claim 10 further comprising an external charging device (300); said external charging device (300) comprising:
- a power supply for supply of the charging power, and
- the transmitter, Tx charging coil (700).

12. The hearing system (150) according to claim 11, wherein the rechargeable battery assembly (104) of the hearing implant (100) comprises a first magnetic component (114) and the external charging device (300) comprises a second magnetic component (314);
- said first and second magnetic components (114, 314)) arranged for providing magnetic attraction and alignment between the receiver, Rx charging coil (650) and the transmitter, Tx charging coil (700).

13. The hearing system according to claim 11 or 12, wherein the external charging device (300) further comprises:
- one or more second microphone(s) (312) for detecting an acoustic signal,
- a third control unit (308) for producing a second digital signal representative of the acoustic signal detected by the one or more second microphone(s) (312), and
- a third radio system (302) for communication with the first radio system (102) of the hearing implant (100), the third radio system (302) being adapted for transmitting the second digital signal to the hearing implant (100).

## Patentansprüche

1. Hörimplantat (100), wobei das Hörimplantat (100) bei einem Benutzer implantierbar ist und Folgendes umfasst:
- ein erstes Funksystem (102), das eine erste magnetische Induktionsspulenantenne (600) zur drahtlosen Kommunikation mit einem externen Hörgerät (200) über eine Nahfeld-Magnetinduktions-Datenverbindung (NFMI) (112) umfasst.
- eine wiederaufladbare Batterieanordnung (104) bestehend aus einer wiederaufladbaren Batterie und einer Empfängerladespule (650) Rx, die zum Laden der wiederaufladbaren Batterie konfiguriert ist, wobei die Empfängerladespule (650) über eine drahtlose magnetische Induktionsverbindung mit einer Senderladespule (700) Tx eines externen Ladegeräts (300) verbunden werden kann, um die Ladeleistung vom externen Ladegerät (300) zu empfangen.
- eine Elektrodenanordnung (106) zur Stimulation eines Hörnervs des Benutzers als Reaktion auf empfangene elektrische Stimulationssignale (510), und
- eine erste Steuereinheit (108), die so konfiguriert ist, dass sie ein erstes digitales Signal über das erste Funksystem (102) empfängt und dieses erste digitale Signal in elektrische Stimulationssignale (510) umwandelt, wobei das erste digitale Signal ein akustisches Signal repräsentiert, wie zum Beispiel ein Audiosignal wie Sprache, das vom externen Hörgerät (200) erfasst und/oder empfangen wird.

2. Das Hörimplantat (100) nach Anspruch 1, wobei die Induktivität der ersten NFMI-Spulenantenne (600) zwischen 2 µH und 20 µH, beispielsweise zwischen 3 µH und 10 µH, liegt.

3. Das Hörimplantat (100) nach Anspruch 1 oder 2, wobei die erste MI-Spulenantenne (600) eine um einen magnetisch permeablen Kern gewickelte Solenoidspule umfasst; und optional ein Volumen von weniger als 20 mm³ aufweist, beispielsweise einen Durchmesser von weniger als 3 mm und eine Länge von weniger als 6 mm.

4. Das Hörimplantat (100) nach Anspruch 2 oder 3, wobei die Induktivität der Rx-Ladespule (650) zwischen 10 µH und 50 µH bei 100 kHz, beispielsweise zwischen 20 µH und 40 µH, liegt.

5. Das Hörimplantat (100) nach Anspruch 4, wobei der Empfänger, die Ladespule Rx (650), eine mehrlagige Luftspule umfasst, die an einen magnetisch permeablen Träger, wie beispielsweise einen flachen kreisförmigen Ferritträger, anliegt .

6. Das Hörimplantat (100) nach einem der vorhergehenden Ansprüche, wobei die erste Steuereinheit (108) einen digitalen Signalprozessor, DSP und/oder einen Mikroprozessor umfasst.

7. Das Hörimplantat (100) nach einem der vorhergehenden Ansprüche, wobei die NFMI-Datenkommunikationsverbindung (112) eine Trägerfrequenz von etwa 5 MHz bis etwa 30 MHz, beispielsweise etwa 10 MHz bis etwa 27 MHz, oder auch etwa 20 MHz bis etwa 25 MHz, beispielsweise 22,6 MHz, umfasst.

8. Ein externes Hörgerät (200) zur Montage am oder im Ohr eines Benutzers, umfassend:
- ein zweites Funksystem (202) zur drahtlosen Kommunikation mit dem ersten Funksystem (102) des Hörimplantats (100) gemäß einem der Ansprüche 1-7 über die NFMI-Datenkommunikationsverbindung (112), wobei das zweite Funksystem (202) eine zweite MI-Spulenantenne umfasst, die in einem Gehäuse des externen Hörgeräts (200) montiert ist,
- eine zweite Steuereinheit (208), die so konfiguriert ist, dass sie das erste digitale Signal erzeugt und über die zweite MI-Spulenantenne an das erste Funksystem (102) des Hörimplantats (100) sendet.

9. Ein externes Hörgerät (200) nach Anspruch 8, umfassend ein Mikrofon (212), das im Inneren des Gehäuses des externen Hörgeräts (200) angeordnet oder mit diesem verbunden ist, zur Aufnahme des akustischen Signals aus der Umgebung des externen Hörgeräts (200).

10. Ein Hörsystem (150), das mindestens Folgendes umfasst:
- das Hörimplantat (100) gemäß einem der Ansprüche 1-7, und
- das externe Hörgerät (200) nach Anspruch 8 oder 9.

11. Das Hörsystem (150) nach Anspruch 10, ferner umfassend eine externe Ladevorrichtung (300); wobei die externe Ladevorrichtung (300) Folgendes umfasst :
- ein Netzteil zur Bereitstellung der Ladeleistung,
- der Sender, Tx Ladespule (700).

12. Das Hörsystem (150) nach Anspruch 11, wobei die wiederaufladbare Batterieeinheit (104) des Hörimplantats (100) ein erstes magnetisches Bauteil (114) und die externe Ladevorrichtung (300) ein zweites magnetisches Bauteil (314) umfasst;
- wobei das erste und das zweite magnetische Bauteil (114, 314) so angeordnet sind, dass sie eine magnetische Anziehung und Ausrichtung zwischen dem Empfänger, der Ladespule (650) Rx und dem Sender, der Ladespule (700) Tx, gewährleisten.

13. Das Hörsystem nach Anspruch 11 oder 12, wobei die externe Ladevorrichtung (300) ferner Folgendes umfasst:
- ein oder mehrere zweite Mikrofone (312) zur Erfassung eines akustischen Signals,
- eine dritte Steuereinheit (308) zur Erzeugung eines zweiten digitalen Signals, das repräsentativ für das von dem/den zweiten Mikrofon(en) (312) erfasste akustische Signal ist, und
- ein drittes Funksystem (302) zur Kommunikation mit dem ersten Funksystem (102) des Hörimplantats (100), wobei das dritte Funksystem (302) für die Übertragung des zweiten digitalen Signals an das Hörimplantat (100) ausgelegt ist.

## Revendications

1. Implant auditif (100), l'implant auditif (100) étant implantable chez un utilisateur et comprenant :
- un premier système radio (102) qui comprend une première antenne à induction magnétique, bobine **MI** (600) pour la communication sans fil avec un dispositif auditif externe (200) via une liaison de communication de données à induction magnétique en champ proche, NFMI (112),
- un ensemble de batterie rechargeable (104) comprenant une batterie rechargeable et un récepteur, bobine de charge Rx (650) configuré pour fournir une puissance de charge à la batterie rechargeable, dans lequel le récepteur, bobine de charge Rx (650) est connectable à un émetteur, bobine de charge Tx (700) d'un dispositif de charge externe (300) via une liaison par induction magnétique sans fil pour recevoir la puissance de charge du dispositif de charge externe (300),
- un réseau d'électrodes (106) pour stimuler un nerf cochléaire de l'utilisateur en réponse à des signaux de stimulation électrique reçus (510), et
- une première unité de commande (108) configurée pour recevoir un premier signal numérique via le premier système radio (102), et pour traiter le premier signal numérique en signaux de stimulation électrique (510), le premier signal numérique étant représentatif d'un signal acoustique, tel qu'un signal audio comme la parole, détecté et/ou reçu par le dispositif auditif externe (200).

2. L'implant auditif (100) selon la revendication 1, dans lequel l'inductance de la première antenne à bobine NFMI (600) est comprise entre 2 µH et 20 µH, par exemple entre 3 µH et 10 µH.

3. L'implant auditif (100) selon la revendication 1 ou 2, dans lequel la première antenne à bobine MI (600) comprend une bobine solénoïde enroulée autour d'un noyau magnétiquement perméable ; et en option un volume inférieur à 20 mm ³ par exemple un diamètre inférieur à 3 mm et une longueur inférieure à 6 mm.

4. L'implant auditif (100) selon la revendication 2 ou 3, dans lequel l'inductance de la bobine de charge Rx (650) est comprise entre 10 µH et 50 µH à 100 kHz, par exemple entre 20 µH et 40 µH.

5. L'implant auditif (100) selon la revendication 4, dans lequel le récepteur, la bobine de charge Rx (650) comprend une bobine d'air multicouche contre un support magnétiquement perméable tel qu'un support circulaire plat en ferrite.

6. L'implant auditif (100) selon l'une quelconque des revendications précédentes, dans lequel la première unité de commande (108) comprend un processeur de signal numérique, DSP et/ou un microprocesseur.

7. L'implant auditif (100) selon l'une quelconque des revendications précédentes, dans lequel la liaison de communication de données NFMI (112) comprend une fréquence porteuse d'environ 5 MHz à environ 30 MHz, telle qu'environ 10 MHz à environ 27 MHz, ou même d'environ 20 MHz à environ 25 MHz, telle que 22,6 MHz.

8. Un dispositif auditif externe (200) destiné à être monté sur ou dans l'oreille d'un utilisateur, comprenant :
- un deuxième système radio (202) pour la communication sans fil avec le premier système radio (102) de l'implant auditif (100) selon l'une quelconque des revendications 1 à 7 via la liaison de communication de données NFMI (112),
ledit deuxième système radio (202) comprenant une deuxième antenne à bobine MI montée à l'intérieur d'un boîtier du dispositif auditif externe (200),
- une deuxième unité de commande (208) configurée pour générer et transmettre le premier signal numérique au premier système radio (102) de l'implant auditif (100) via la deuxième antenne à bobine MI.

9. Un dispositif auditif externe (200) selon la revendication 8, comprenant un microphone (212) disposé à l'intérieur du boîtier du dispositif auditif externe (200), ou connectable au dispositif auditif externe (200), pour capter le signal acoustique de l'environnement du dispositif auditif externe (200).

10. Un système auditif (150) comprenant au moins :
- l'implant auditif (100) selon l'une quelconque des revendications 1 à 7, et
- le dispositif auditif externe (200) selon la revendication 8 ou 9.

11. Le système auditif (150) selon la revendication 10 comprend en outre un dispositif de charge externe (300) ; ledit dispositif de charge externe (300) comprenant :
- une alimentation électrique pour fournir l'énergie de charge, et
- l'émetteur, bobine de charge Tx (700).

12. Le système auditif (150) selon la revendication 11, dans lequel l'ensemble de batterie rechargeable (104) de l'implant auditif (100) comprend un premier composant magnétique (114) et le dispositif de charge externe (300) comprend un deuxième composant magnétique (314) ;
- lesdits premier et deuxième composants magnétiques (114, 314) disposés pour assurer l'attraction magnétique et l'alignement entre le récepteur, bobine de charge Rx (650) et l'émetteur, bobine de charge Tx (700).

13. Le système auditif selon la revendication 11 ou 12, dans lequel le dispositif de charge externe (300) comprend en outre :
- un ou plusieurs deuxièmes microphones (312) pour détecter un signal acoustique,
- une troisième unité de commande (308) pour produire un deuxième signal numérique représentatif du signal acoustique détecté par le ou les deuxièmes microphones (312), et
- un troisième système radio (302) pour la communication avec le premier système radio (102) de l'implant auditif (100), le troisième système radio (302) étant adapté pour transmettre le deuxième signal numérique à l'implant auditif (100).
